## (19) European Patent Office

Europäisches Patentamt

European Patent Office

Office européen des brevets

(11) **EP 1 156 996 B1**

## (12) EUROPEAN PATENT SPECIFICATION

(45) Date of publication and mention
of the grant of the patent:
**01.09.2004 Bulletin 2004/36**

(21) Application number: **00904128.6**

(22) Date of filing: **02.02.2000**

(51) Int Cl.$^7$: **C07C 63/08**, B01J 2/12

(86) International application number:
**PCT/NL2000/000066**

(87) International publication number:
**WO 2000/047543 (17.08.2000 Gazette 2000/33)**

(54) **PROCESS FOR THE SHAPING OF ALKALINE (EARTH) METAL BENZOATE PARTICLES**

VERFAHREN ZUM FORMEN VON (ERD)ALKALIMETAL BENZOATPARTIKELN

PROCEDE DE MISE EN FORME DE PARTICULES DE BENZOATE DE METAUX ALCALINO (TERREUX)

(84) Designated Contracting States:
**AT BE CH CY DE DK ES FI FR GB GR IE IT LI LU MC NL PT SE**

(30) Priority: **15.02.1999 NL 1011307**

(43) Date of publication of application:
**28.11.2001 Bulletin 2001/48**

(73) Proprietor: **DSM IP Assets B.V.**
**6411 TE Heerlen (NL)**

(72) Inventors:
• **STOELWINDER, Christiaan, Johannes, Cornelis**
**NL-6137 LM Sittard (NL)**
• **ROZIE, Hendricus, Johannes**
**NL-6123 BB Holtum (NL)**
• **KLEIN VELDERMAN, Johan, Christiaan**
**NL-2805 AS Gouda (NL)**

(74) Representative: **Jacobs, Monique S.N.**
**DSM Intellectual Property**
**Office Geleen**
**P.O. Box 9**
**6160 MA Geleen (NL)**

(56) References cited:
**FR-A- 2 705 677          US-A- 5 276 000**

• **DATABASE WPI Section Ch, Week 198918 Derwent Publications Ltd., London, GB; Class B05, AN 1989-136236 XP002137127 & JP 01 083044 A (TOA GOSEI CHEM IND LTD), 28 March 1989 (1989-03-28) cited in the application**
• **DATABASE WPI Section Ch, Week 198733 Derwent Publications Ltd., London, GB; Class D22, AN 1987-233237 XP002137128 & NL 8 503 102 A (STAMICARBON BV), 1 June 1987 (1987-06-01)**

**Description**

The invention relates to a process for the shaping of alkaline (earth) metal benzoate particles.

From JP-A-01083044 in the name of Toa Gosei it is known to extrude a sodium benzoate paste, which contains 20-30 wt.% $H_2O$, to form a granulate with a diameter of 0.2-1.2 mm, following which the granules are dried until the amount of water is less than 1 wt.%.

However, the known sodium benzoate granules are found to be highly smearable, as a consequence of which clogging readily takes place in, for instance, processing equipment used in the treatment and transport of these granules. Upon drying and processing of the sodium benzoate granules, moreover, dust formation generally occurs, which is undesirable from the point of view of good occupational hygiene and from the point of view of good process efficiency. The reason for this is that the texture of the granulate formed is so weak that much dust is formed during subsequent handling.

Alkaline (earth) metal benzoates are mostly used as preservatives in foodstuffs, for instance in soft drinks.

The aim of the present invention is to find a solution for the above-mentioned drawbacks.

This aim is achieved in that the shaping process comprises a process step in which the alkaline (earth) metal benzoate particles make a spinning motion involves the particles being subjected to centrifugal forces, so that the particles which motion revolve around their own axis.

It has been found that when alkaline (earth) metal benzoate particles are subjected to such a process step their smearability is substantially reduced. It has also been found according to the invention that no significant dust formation occurs either during drying or during processing of the granules.

In FR-A-2 705 677 a process is disclosed for extruding-spheronizing cyclodextrine particles in order to improve the particle shape, particle size distribution, abrasion resistance and flow properties.

Examples of the alkaline (earth) metal benzoate particles to which the process according to the invention applies are particles obtained via a granulation technique, for instance fluid bed granulation, palletizing, steam granulation, wet granulation or pan granulation and particles obtained via an extrusion technique, for instance high-pressure extrusion and low pressure extrusion. Such granulation and extrusion techniques are generally known. Examples of suitable extruders are single-screw extruders and twin-screw extruders, both with co-rotating and with counterrotating screws. The extrudate formed can be removed from the extruder both axially and radially.

The alkaline (earth) metal benzoate particles are for instance formed from an alkaline earth metal benzoate powder, which to that end is optionally mixed with a liquid, preferably water. The powder and the liquid can be mixed either before the granulation or extrusion process or during the extrusion process. Suitable mixing equipment is known to one skilled in the art. During the extrusion process mixing can suitably take place by the use of suitable mixing elements, in the extruder.

Preferably, the alkaline (earth) metal benzoate particles are obtained from a paste of alkaline (earth) metal benzoate and water, which is for instance extruded in an axial or radial extruder to form alkaline (earth) metal benzoate extrudate.

After passing through a so-called die, mould or screen, the extrudate produced can optionally be cut or chopped to a certain defined length, but it is also well possible not to define the length of the extrudate in such a way and allow the extrudate to flow from the extruder in long strands. The extrudate for instance has a cylindrical shape and its diameter can be chosen within wide limits. The diameter generally lies between 0.1 and 10 mm, preferably between 0.2 and 5 mm, more preferably between 0.5 and 3 mm.

The alkaline (earth) metal benzoate for instance is sodium, potassium, calcium and/or magnesium benzoate. Preferably, it is sodium benzoate. The alkaline (earth) metal particles may optionally contain additives.

The water content of the alkaline (earth) metal benzoate particles is chosen so that the particles are plastically deformable, while furthermore they are not sticky and do not adhere, so that problems such as clogging can be minimized in the shaping step. The water content of the alkaline (earth) metal benzoate particles that are subjected to the shaping step according to the invention amounts to 5-40 wt.% water, preferably 10-30 wt.% water, in particular 15-25 wt.% water.

In the shaping process according to the invention the alkaline (earth) metal benzoate particles are subjected to a process step in which the alkaline (earth) metal benzoate particles make a spinning motion. In the framework of the invention a spinning motion is understood to mean that the alkaline (earth) metal benzoate particles make a motion which involves them being subjected to, for instance, centrifugal forces, so that the particles revolve around their own axis.

Preferably, the above-mentioned process step is carried out in a spheronizer. An example of a suitable apparatus is a spheronizer or a marumerizer® as described in US-A-3277520. A marumerizer® is an apparatus with a side wall that is optionally smooth, for instance a cylinder with a rotatable, essentially horizontal plate placed concentrically in it. The plate, if desired provided with a profile or roughened, can rotate around an essentially vertical shaft.

If desired the plate's surface is provided with a profile or structure, depending on the process to be used. The plate is placed at a small distance from the side walls.

In this apparatus the extrudate falls onto the rotating plate, upon which the particles or strands are gradually transformed into particles of which the length/diameter ratio varies between, for instance, 1 and 2.5. The particles or strands can be fed to the spheronizer directly from the granulator or extruder, but it is also well possible, for instance, to first dry the granulate or extrudate and subsequently if desired moisten it before it is supplied to the spheronizer.

If desired the spheronizer may contain various plates and/or two or more apparatuses can be placed in series to realize a plug flow character. In this way the spread in residence time of the individual particles is reduced.

The motion of the particles can essentially be described as follows. The particles are forced against the wall of the apparatus, a combination of gravity, centrifugal forces and friction forces resulting in the formation of a mechanically fluidized ring of particles that slowly revolves relative to the wall.

The individual particles follow a helix-shaped course, with the particles being plastically deformed and gradually changing their shape from, for instance, cylindrical via dumbbell-shaped to spherical. The final shape is the result of the various process parameters and is affected, for instance, by the residence time, the speed of the plate and the moisture content of the particles. The process step can be carried out batchwise, semi-continuously or continuously.

The duration of the shaping of the alkaline (earth) metal benzoate particles depends on the desired shape of the product. At a longer residence time in the spheronizer the alkaline (earth) metal benzoate particles, for instance cylindrical rods, will further be converted, via cylinders with rounded ends or dumbbell-shaped particles, to elliptical particles, and ultimately spherical alkaline (earth) metal benzoate particles are obtained. The duration of shaping may for instance vary from 5 seconds to 30 minutes. Preferably, the average duration is 10-300 seconds. The duration of shaping that is chosen results in an optimum combination of the desired particle shape and the desired shaping capacity.

The speed of the rotating plate may vary within wide limits and for instance lies between 100-5000 revolutions/minute. Preferably, the speed lies between 300-1000 revolutions/minute, in particular between 500-700 revolutions/minute.

The desired process conditions depend on the specific situation and can simply be determined by one skilled in the art.

The temperature of the alkaline (earth) metal benzoate particles to be used may vary between wide limits, for instance between 20-120°C. It is, for instance, possible to spheronize alkaline (earth) metal benzoate particles that come directly from an extruder. The temperature, however, is not critical during the shaping process and one skilled in the art will simply be able to optimize the other parameters at any temperature.

The invention also relates to alkaline (earth) metal benzoate particles having a low smearability. Such particles can for instance be obtained by the process according to the invention.

The smearability is important in further processing of the alkaline (earth) metal benzoate particles; particles with a lower smearability will less often cause clogging and/or cause less dust formation when subjected to treatments such as, for instance, drying, transport, for instance via a conveyor screw or via pneumatic transport, packaging, metering and during storage.

The smearability is measured analogously to ASTM C142-78, the particles being rolled/smeared/squeezed individually between thumb and forefinger. The particle should not come into contact with the nail or other hard surfaces, and the fingers must be dry. Then the number of smearable particles is counted, that is, how many particles remain intact and how many do not. Lastly, the percentage of the number of smearable particles is calculated. In practice, on normal execution of the test, between 50 and 95% of the non-spheronized particles will be smearable; of the spheronized particles the percentage of smearable particles will be at least a factor 1.5, preferably a factor 5, in particular a factor 10, lower than the value found for the particles obtained before spheronizing.

The smearability of the shaped, spheronized alkaline (earth) metal benzoate particles is as a rule lower than 50%. Preferably, the smearability is lower than 40%, in particular lower than 10%.

It has been found that other mechanical properties of the alkaline (earth) metal benzoate particles, too, improve significantly, and that the product obtained contained essentially no vacuoles.

The shaped alkaline (earth) metal benzoate particles have a crushability lower than 40%. The crushability is a measure of the hardness of the particles and is measured by means of the so-called ball test. In this test 25 g of the shaped particles product is introduced, together with 18 steel balls (diameter 15 mm), into a sieve pan and placed on a shaking screen. The shaking screen must perform a circular movement in the horizontal plane at a frequency of about 250 revolutions per minute and an amplitude (top to top) of 15 mm. The mesh width of the shaking screen depends on the product to be used and can readily be chosen by one skilled in the art. If the diameter of the particles to be used is larger than 0.6 mm, use is made of a shaking screen having a mesh width of 0.5 mm. The weight percentage of the particles smaller than 0.5 mm relative to the amount of starting material (25 gram) is the crushability.

Preferably, the crushability of the alkaline (earth) metal benzoate particles is smaller than 20%.

It has been found that also the bulk density of the shaped alkaline (earth) metal benzoate particles has improved. The shaped alkaline (earth) metal benzoate particles usually have a bulk density in excess of 550 kg/m$^3$. Preferably, the bulk density is higher than 600 kg/m$^3$, in particular higher than 650 kg/m$^3$. Alkaline (earth) metal benzoate particles with a high bulk density are highly desirable form a packaging point of view. Stacks of bags filled with product having a high bulk density are stabler and less inclined to sag and/or collapse and are consequently safer.

The shaped alkaline (earth) metal benzoate particles usually have the shape of a sphere and/or dumbbell. The shape of the particles is important in view of the flow properties. Spherical and/or dumbbell-shaped alkaline (earth) metal benzoate particles exhibit better flow properties, so that transport of these particles causes fewer problems.

The alkaline (earth) metal benzoate particles according to the invention for instance have a length / diameter ratio lower than 2.5. Preferably, the length / diameter ratio is lower than 1.5.

Spheronizing has no or hardly any effect on the porosity of the spheronized alkaline (earth) metal benzoate particles compared with, for instance, non-spheronized alkaline metal extrudate. It is surprising that the smearability and the crushability should improve strongly, while the porosity of the particles shows no or hardly any change. Surprisingly, the effect on the dissolution rate, too, is small.

The alkaline (earth) metal benzoate particles are, for instance, potassium, sodium, calcium and/or magnesium benzoate particles, preferably sodium benzoate particles.

The invention will now be elucidated on the basis of the examples, without being restricted to these.

Example I

2250 g sodium benzoate powder and 750 g water were mixed intensively for 155 seconds until a homogeneous, non-sticky paste was formed.

The resulting paste was fed to an extruder (Fuji Paudal, type EXDF-100), which was equipped with a die plate with 2 mm diameter holes. The speed of the screws was 36 revolutions per minute and long strands of extrudate were obtained.

The resulting extrudate was fed to a spheronizer (Fuji Paudal marumerizer®, type QJ-400), which was provided with a profiled bottom plate (plate pitch 3 mm). The speed of the bottom plate was 630 revolutions per minute and the duration of the spheronizing process was 67 seconds. A good, fluidized band was obtained, and after the process dumbbell-shaped particles were obtained, of which the average length/diameter ratio was between 1.5 and 2.5. After drying (15 minutes at 100°C in a fluid bed dryer) a product having a bulk density of 694 kg/m$^3$ was obtained.

The smearability of the particles was determined by rolling / squeezing / smearing 100 particles individually between thumb and forefinger, without the particles coming into contact with nails or hard surfaces. Of the 100 particles, 4 were smearable.

The crushability of the product was measured using the ball test described above and amounted to 15.6 wt.%.

To measure the strength of the product, 100 particles (average length 3.1 mm) were fed via a vibrating chute to a tube inside of which an air velocity of 20 m/s was created. The particles were shot against a plate placed at an angle of 45°C relative to the longitudinal direction of the tube. Upon completion of the experiment the average length was 3.1 mm. The product obtained did not contain any vacuoles.

Example II

Analogously to example I 6750 g sodium benzoate powder and 2250 g water were mixed for 300 seconds.

The resulting paste was fed at room temperature to a radial extruder (Fuji Paudal, type EXDCS), which was equipped with a screen with holes of 1 mm diameter (screw speed 54 revolutions per minute). Long extrudate was formed.

The resulting extrudate was fed to a spheronizer (QJ-400, plate pitch 3 mm, 25 seconds, 630 revolutions per minute, good fluidization).

The average length / diameter ratio of the dumbbell-shaped product was between 1.5 and 2.5. After drying, the bulk density was 611 kg/m$^3$. Analogously to example I the smearability (3%), the crushability (12.3 wt.%) and the strength (before: 2.4 mm, after: 2.3 mm) were determined. The product obtained did not contain any vacuoles.

Comparative Experiment A

In conformity with example II, the smearability (93%), the crushability (55.8 wt.%) and the strength (before: 3.2 mm, after: 2.3 mm) of the extrudate obtained via the process according to example II (bulk density 525 kg/m$^3$) II were determined after drying.

EP 1 156 996 B1

TABLE 1

| Comparison of various properties | | | |
|---|---|---|---|
| | | Ex.II | Exp.A |
| Smearability (%) | | 3 | 93 |
| Crushability (wt.%) | | 12.3 | 55.8 |
| Strength: | Length before (mm) | 2.4 | 3.2 |
| | Length after (mm) | 2.3 | 2.3 |
| Internal porosity, (%) | 1 | 24 | 29 |
| Dissolving rate, sec. 10% wt. product in water | 2 | 50 | 35 |
| Settling angle, °(150 ml prod. height 75 mm) | 3 | 31.9 | 35.0 |
| Collapsing angle, ° | 4 | 38.1 | 42.6 |
| Compressibility, % | 5 | 12 | 10.3 |
| Apparent density, kg/m$^3$ | 6 | 696 | 563 |

1. Internal porosity = $\frac{\text{empty pore volume}}{\text{total volume}}$ x 100% Total volume = empty pore volume + volume of the solid material. The empty pore volume is determined via mercury porosimetry. The volume of the solid material is measured by means of the helium test.

2. The dissolving rate is the dissolution time in seconds that is needed for dissolving 10 wt.% of product in water at 20°C.

3. The settling angle is measured in conformity to DIN ISO 4324.

4. The collapsing angle is the minimal slope of the base, in °, at which the product slides downward.

5. Compressibility = (apparent density-bulk density)/ apparent density x 100%

6. The apparent density was measured using a standard test method, ASTM D 1895 -89 (method A).

**Claims**

1. Process for the shaping of alkaline (earth) metal benzoate particles, **characterized in that** the shaping process comprises a step in which the alkaline (earth) metal benzoate particles make a spinning motion, which motion invokes the particles being subjected to centrifugal forces, so that the particles revolve around their own axis.

2. Process according to claim 1, **characterized in that** the alkaline (earth) metal benzoate particles are alkaline (earth) metal benzoate extrudate.

3. Process according to claim 1 or claim 2, **characterized in that** the alkaline (earth) metal benzoate partides contain 5-40 wt.% $H_2O$.

4. Process according to any one of claims 1-3, **characterized in that** the average duration of the shaping step is at least 5 seconds.

5. Process according to any one of claims 1-4, **characterized in that** shaping takes place in a spheronizer.

6. Process according to any of claims 1-5, **characterized in that** the alkaline (earth) metal benzoate particles are sodium benzoate particles.

7. Alkaline (earth) metal benzoate particles, **characterized in that** the smearability is lower than 50%.

8. Alkaline (earth) metal benzoate particles according to claim 7, **characterized in that** the smearability is lower than 40%, preferably 10%.

9. Alkaline (earth) metal benzoate particles according to claim 7 or claim 8, **characterizing in that** the crushability is lower than 40%.

10. Alkaline (earth) metal benzoate particles according to any one of claims 7-9, **characterized in that** the average length / diameter ratio of the alkaline (earth) metal benzoate particles is lower than 2.5.

11. Alkaline (earth) metal benzoate partides according to any one of claims 7-10, **characterized in that** the particles have a spherical and/or dumbbell shape.

12. Alkaline (earth) metal benzoate particles according to any one of claims 7-11, **characterized in that** the alkaline (earth) metal benzoate particles have a bulk density higher than 550 kg/m$^3$.

13. Alkaline (earth) metal benzoate particles according to any one of claims 7-12, **characterized in that** they are sodium benzoate particles.

**Patentansprüche**

1. Verfahren zur Formgebung von (Erd)-Alkalimetallbenzoat-Partikeln, **dadurch gekennzeichnet, dass** das Formgebungsverfahren einen Schritt umfasst, bei'welchem die (Erd)-Alkalimetallbenzoat-Partikel eine Drehbewegung machen, welche Bewegung einbezieht, dass die Partikel Zentrifugalkräften unterworfen werden, so dass sich die Partikel um ihre eigene Achse drehen.

2. Verfahren gemäß Anspruch 1, **dadurch gekennzeichnet, dass** die (Erd)-Alkalimetallbenzoat-Partikeln ein (Erd)-Alkalimetallbenzoat-Extrudat sind.

3. Verfahren gemäß Anspruch 1 oder Anspruch 2, **dadurch gekennzeichnet, dass** die (Erd)-Alkalimetallbenzoat-Partikel 5-40 Gew.-% H$_2$O enthalten.

4. verfahren gemäß einem der Ansprüche 1-3, **dadurch gekennzeichnet, dass** die durchschnittliche Dauer des Formgebungsschritts mindestens 5 Sekunden beträgt.

5. Verfahren gemäß einem der Ansprüche 1-4, **dadurch gekennzeichnet, dass** die Formgebung in einem Spheronizer stattfindet.

6. Verfahren gemäß einem der Ansprüche 1-5, **dadurch gekennzeichnet, dass** die (Erd)-Alkalimetallbenzoat-Partikel Natriumbenzoat-Partikel sind.

7. (Erd)-Alkalimetallbenzoat-Partikel, **dadurch gekennzeichnet, dass** die Schmierfähigkeit kleiner als 50% ist.

8. (Erd)-Alkalimetallbenzoat-Partikel gemäß Anspruch 7, **dadurch gekennzeichnet, dass** die Schmierfähigkeit kleiner als 40%, vorzugsweise 10% ist.

9. (Erd)-Alkalimetallbenzoat-Partikel gemäß Anspruch 7 oder Anspruch 8, **dadurch gekennzeichnet, dass** die Brechbarkeit kleiner als 40% ist.

10. (Erd)-Alkalimetallbenzoat-Partikel gemäß einem der Ansprüche 7-9, **dadurch gekennzeichnet, dass** das durchschnittliche Länge/Durchmesser-Verhältnis der (Erd)-Alkalimetallbenzoat-Partikel kleiner als 2,5 ist.

11. (Erd)-Alkalimetallbenzoat-Partikel gemäß einem der Ansprüche 7-10, **dadurch gekennzeichnet, dass** die Partikel eine Kugelund/oder Hantelform haben.

12. (Erd)-Alkalimetallbenzoat-Partikel gemäß einem der Ansprüche 7-11, **dadurch gekennzeichnet, dass** die (Erd)-Alkalimetallbenzoat-Partikel eine Schüttdichte höher als 55 kg/m$^3$ haben.

13. (Erd)-Alkalimetallbenzoat-Partikel gemäß einem der Ansprüche 7-12, **dadurch gekennzeichnet, dass** sie Natriumbenzoat-Partikel sind.

**Revendications**

1. Procédé de mise en forme de particules de benzoate de métal alcalin (terreux), **caractérisé en ce que** le procédé de mise en forme comprend une étape dans laquelle les particules de benzoate de métal alcalin (terreux) effectuent un mouvement tournant, ce mouvement impliquant que les particules sont soumises à des forces centrifuges de sorte que les particules tournent autour de leur propre axe.

2. Procédé selon la revendication 1, **caractérisé en ce que** les particules de benzoate de métal alcalin (terreux) sont le produit d'extrusion de benzoate de métal alcalin (terreux).

3. Procédé selon la revendication 1 ou 2, **caractérisé en ce que** les particules de benzoate de métal alcalin (terreux) contiennent 5 à 40% en poids de $H_2O$.

4. Procédé selon l'une quelconque des revendications 1 à 3, **caractérisé en ce que** la durée moyenne de l'étape de mise en forme est d'au moins 5 secondes.

5. Procédé selon l'une quelconque des revendications 1 à 4, **caractérisé en ce que** la mise en forme a lieu dans un sphéroniseur.

6. Procédé selon l'une quelconque des revendications 1 à 5, **caractérisé en ce que** les particules de benzoate de métal alcalin (terreux) sont des particules de benzoate de sodium.

7. Particules de benzoate de métal alcalin (terreux), **caractérisées en ce que** la capacité de graissage est inférieure à 50%

8. Particules de benzoate de métal alcalin (terreux) selon la revendication 7, **caractérisées en** la capacité de graissage est inférieure à 40%, de préférence 10%.

9. Particules de benzoate de métal alcalin (terreux) selon la revendication 7 ou 8, **caractérisées en ce que** l'aptitude au broyage est inférieure à 40%.

10. Particules de benzoate de métal alcalin (terreux) selon l'une quelconque des revendications 7 à 9, **caractérisées en ce que** le rapport moyen de longueur/diamètre des particules de benzoate de métal alcalin (terreux) est inférieur à 2,5.

11. Particules de benzoate de métal alcalin (terreux) selon l'une quelconque des revendications 7 à 10, **caractérisées en ce que** les particules ont une forme sphérique et/ou une forme d'haltère.

12. Particules de benzoate de métal alcalin (terreux) selon l'une quelconque des revendications 7 à 11, **caractérisées en ce que** les particules de benzoate de métal alcalin (terreux) ont une masse volumique supérieure à 550 kg/m$^3$.

13. Particules de benzoate de métal alcalin (terreux) selon l'une quelconque des revendications 7 à 12, **caractérisées en ce qu'**elles sont des particules de benzoate de sodium.